# EUROPEAN PATENT SPECIFICATION

(11) **EP 3 302 268 B1**
(45) Date of publication and mention of the grant of the patent: **17.11.2021**
(21) Application number: 16820605.0
(22) Date of filing: 06.07.2016
(51) Int. Cl.: A61B 34/20, A61B 5/00, A61B 5/107, A61B 90/00

(54) **LEG LENGTH AND OFFSET CALCULATION IN COMPUTER-ASSISTED SURGERY USING RANGEFINDER**
BEINLÄNGEN- UND OFFSET-BERECHNUNG BEI DER COMPUTERGESTÜTZTEN CHIRURGIE UNTER VERWENDUNG EINES ENTFERNUNGSMESSERS
CALCUL DE LONGUEUR DE JAMBE ET DE DÉCALAGE EN CHIRURGIE ASSISTÉE PAR ORDINATEUR À L'AIDE D'UN TÉLÉMÈTRE

(30) Priority: 06.07.2015 US 201562188921 P; 24.03.2016 US 201662312509 P
(43) Date of publication of application: 11.04.2018
(73) Proprietor: Orthosoft ULC, Montréal, QC H3C 2N6 (CA)
(72) Inventor: LI, Di, LaSalle, Québec H8N 3G9 (CA); AMIOT, Louis-Philippe, Hampstead, Québec H3X 3G8 (CA); PARADIS, François, Boucherville, Québec J4B 7V1 (CA); PELLETIER, Benoit, Montréal, Québec H2C 2L7 (CA); MOREAU-BELANGER, Laurence, Laval, Québec H7M 4J1 (CA); DUVAL, Karine, Montréal, Québec H2K 1E7 (CA)
(74) Representative: Taor, Simon Edward William
(86) International application number: PCT/CA2016/050787
(87) International publication number: WO 2017/004714

(56) References cited:
- US-A1- 2009 125 117
- US-A1- 2010 076 505
- US-A1- 2011 112 589
- US-A1- 2012 157 887
- US-B2- 7 780 681
- US-B2- 8 265 790
- HE ET AL.: 'An Inertial and Optical Sensor Fusion Approach for Six Degree-of-Freedom Pose Estimation' SENSORS, [Online] vol. 15, no. 7, 2015, ISSN 1424-8220 pages 16448 - 16465, XP055315186 ISSN: 1424-8220 Retrieved from the Internet: <URL:www.mdpi.com/journal/sensors>

## Description

### CROSS-REFERENCE TO RELATED APPLICATIONS

The present disclosure claims the priority of US Patent Application Serial No. 62/188,921, filed on July 6, 2015, and the priority of US Patent Application Serial No. 62/312,509, filed on March 24, 2016.

### TECHNICAL FIELD

The present disclosure relates to a system and method used in Computer-Assisted Surgery (CAS) to provide length measurement, such as for leg length discrepancy and offset measurements, for instance in hip surgeries.

### BACKGROUND OF THE ART

Leg length discrepancy is a change of leg length along the longitudinal axis of the patient, following orthopedic surgery. Offset is the measurement of the translational shift of the leg along a medio-lateral axis of the patient. Both these parameters are relevant during hip surgery, including total hip replacement, acetabular cup implanting, femoral implanting (e.g., head and neck implant, resurfacing). There is a need for systems and methods for determining leg length discrepancy and offset for example.

US2010076505 discloses hip preparation systems and methods which can include a surgical orientation device. The hip preparation systems and methods can be used, for example, to orient the hip during the procedure, determine the orientation of an anatomical plane or planes, and orient a prosthetic component or components. Leg length and joint off-set changes can be measured and displayed to a user.

### SUMMARY

It is aim of the present disclosure to provide novel systems and methods for measuring anatomic lengths in computer-assisted orthopedic surgery.

It is another aim of the present disclosure that the lengths measured are used in leg length discrepancy and offset calculations to assess orthopedic hip surgery.

According to the invention, there is provided a system for measuring a length variation between body portions in computer-assisted surgery between a preoperative condition and an intra- or post-operative condition as set out in claim 1

Optional features are set out in the dependent claims.

The feature or features of one embodiment may be applied to other embodiments, even though not described or illustrated, unless expressly prohibited by this disclosure or the nature of the embodiments.

Some details associated with the present embodiments are described above and others are described below.

### DESCRIPTION OF THE DRAWINGS

Fig. 1 is a block diagram of a system for measuring leg length and offset in computer-assisted surgery, in accordance with the present disclosure;
Fig. 2 is a perspective view of a rangefinder apparatus of the system of Fig. 1, in accordance with an embodiment;
Fig. 3 is a perspective view of the rangefinder apparatus of Fig. 2, as mounted to a tripod;
Fig. 4 is a perspective view of the rangefinder apparatus of Fig. 2, as mounted to a base for being mounted onto a patient;
Fig. 5 is a perspective view of the rangefinder apparatus of Fig. 4, as mounted onto a patient, for measuring leg length discrepancy;
Fig. 6 is a perspective view of the rangefinder apparatus of Fig. 4, for measuring both leg length discrepancy and offset; and
Fig. 7 is a schematic view of a geometric arrangement of landmarks used in measuring leg length discrepancy and offset with the system of Fig. 1.

### DETAILED DESCRIPTION

In the proposed disclosure, the leg length discrepancy and offset measurements are resolved using three-dimensional trigonometry.

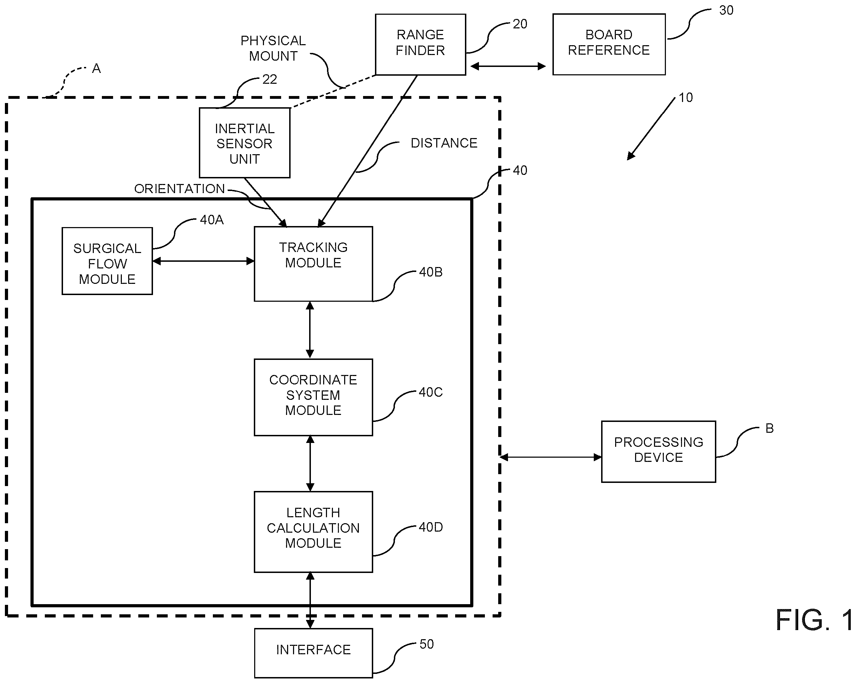

Referring to Fig. 1, a system 10 for measuring leg length in computer-assisted surgery is generally shown as having a rangefinder apparatus 20 (supporting an inertial sensor unit comprising gyroscopes and possibly accelerometers as shown after), an optional board reference device 30, and a computer-assisted surgery (CAS) processor unit 40.

The rangefinder apparatus 20 is of the type having the capacity of outputting distance measurements of a light point it emits on a landmark. The rangefinder apparatus 20 may also be trackable in orientation (pitch, roll and/or yaw) via an inertial sensor unit, optical tracking or equivalent, and possibly in position (X, Y, Z), for instance using optical tracking.

The board reference device 30 may optionally be used to assist in taking the measurements. The board reference device 30 may be a target anchored to the body to serve as an immovable landmark for range measurement.

The CAS processor unit 40 is of the type having a processor and modules adapted to perform calculations to assist an operator during surgery. Accordingly, the CAS processor unit 40 may integrally have or be interfaced to any appropriate interface 50 enabling interaction with the operator, such as a screen (e.g., touchscreen, a display, a monitor, a mouse, keyboard, stylus, etc). While the description provided below refers to the calculation of leg length discrepancy and offset, the CAS processor unit 40 may be used to perform various other functions, with the calculator of leg length discrepancy and offset being one of numerous modules of the system. Alternatively, the system described herein may have the CAS processor unit 40 solely dedicated to the functions described hereinafter of calculating leg length and offset.

Referring to Figs. 1-3, the rangefinder apparatus 20 comprises a rangefinder 21 that is devised to take accurate distance measurement (e.g., accuracy ±1 mm). For example, the rangefinder 21 may be a laser rangefinder 21, emitting a light point and measuring the distance to the light point with the aforementioned accuracy. One possible rangefinder that may be used is the Leica DISTO E7400x rangefinder, provided strictly as an example. As in the illustrated embodiment, the rangefinder apparatus 20 may have a display screen, and may even incorporate the CAS processor unit 40. The rangefinder 21 may alternatively be wired or in wireless communication with the CAS processor unit 40, so as to transmit measurements thereto.

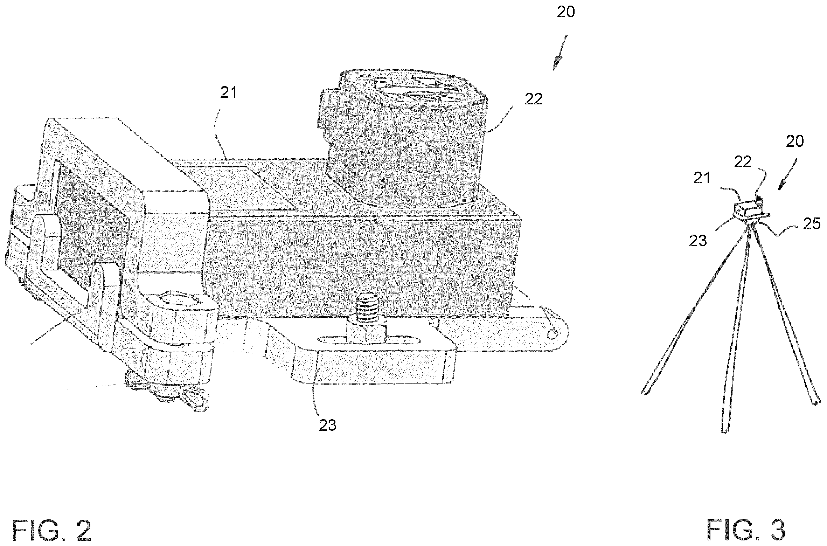

In the methods described hereinafter, three-dimensional (3D) distance and angular measurements are derived from the laser rangefinder 21 and the inertial sensors within the apparatus 20. The rangefinder apparatus 20 therefore has an inertial sensor unit 22 (e.g. iASSIST POD™ including a tri-axial gyroscope, among other components). The apparatus 20 may be sterile or non-sterile (as detailed hereinafter, it is contemplated to keep the apparatus 20 outside of the sterile zone throughout surgery in the embodiment of Fig. 3), and no preoperative imaging is needed for leg length discrepancy and offset measurements. The laser rangefinder 21 and the inertial sensor unit 22 may be interconnected rigidly, such that there is no relative movement between the two, whereby measurements from the inertial sensor unit 22 are indicative of the orientation of the rangefinder 21. The inertial sensor unit 22 may be designed to be connected in a single possible orientation to the rangefinder 21, such that the orientation of the inertial sensor unit 22 is known relative to the rangefinder 21 to which it is connected when turned on.

According to an embodiment, the laser rangefinder 21 and the inertial sensor unit 22 may be operatively supported by a support 23 that is the headplate of a tripod 24, so as to limit movement of the laser rangefinder 21 to three degrees of freedom (DOFs) of rotation (pitch and yaw, possibly roll as well) only (no translational movement), the laser rangefinder 21 and the inertial sensor unit 22 being rigidly connected to the support 23 to form an integral assembly with its components moving concurrently. As shown in Fig. 3, the support 23 is mounted to the tripod 24 by way of a spherical joint 25. Accordingly, the rangefinder 21 is displaceable in orientation (roll, pitch and yaw), but is fixed in position. Other supporting mechanisms may be used to achieve this, although the tripod 24 is a straightforward and readily available solution. It is also contemplated to provide positional tracking to the rangefinder apparatus 20 (e.g., passive optical tracking), to allow free manipulations of the laser rangefinder 21, instead of limiting same to rotational movements only. However, due to the simplicity of the movement constraint shown with the tripod 24, the disclosure will not elaborate on the positional tracking, although the same geometrical calculations may apply to a system with positional tracking capability.

By being movable, the rangefinder apparatus 20 may point to directly selected landmarks without using any board device, depending on the application. For example, given subcutaneous landmarks may be marked (e.g., marker, sticker) or used due to their visual distinctiveness, throughout leg length calculations, as the rangefinder apparatus 20 with three rotational DOFs can be freely oriented to aim at these landmarks, provided there is a direct line of sight between the patient and the rangefinder 21. The change in orientation will be determined by the inertial sensor unit 22 such that, as the beam illuminates the selected landmarks and a distance is measure, the instant 3D orientation of the rangefinder 21 is recorded. The distance measurement provided by the laser rangefinder 21 is combined to the 3D orientation of the apparatus 20, to perform the trigonometric calculations to obtain leg length discrepancy and/or offset, among other possibilities, as described hereinafter.

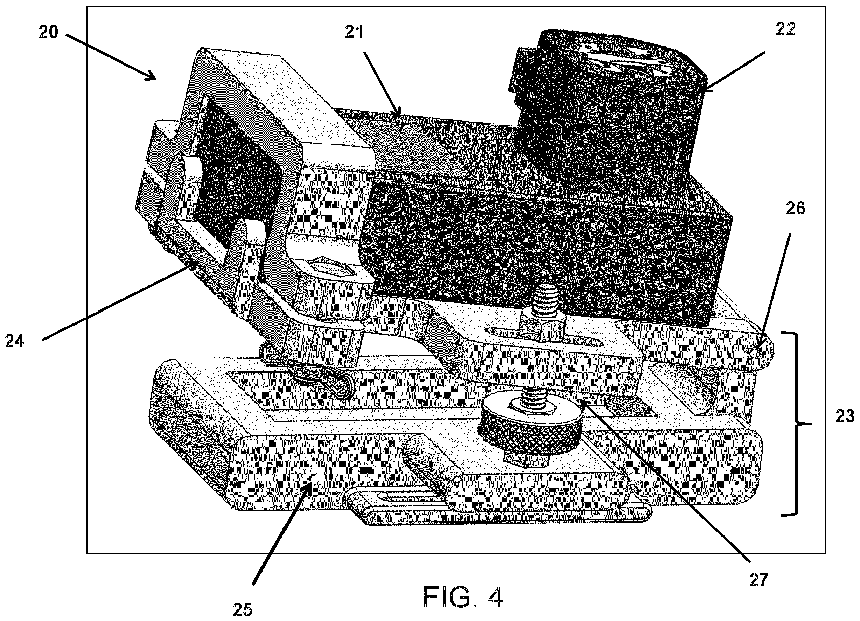

As another arrangement, shown in Fig. 4, the rangefinder apparatus 20 is mounted to a base that is secured to the patient. Its inertial sensor unit 22 may include accelerometers to project the distance and angular measurements into the patient frontal plane, which may be assumed to be the plane of the operating room (OR) table A when the OR table is leveled. The leg length discrepancy and offset measurements may be resolved based on this assumption, as described hereinafter, using trigonometry.

In the embodiment of Fig. 4, the laser rangefinder 21 and the inertial sensor unit 22 may be operatively supported by the support 23 providing a pitch degree of freedom (DOF) of rotation. In Fig. 3, the support 23 is shown as having a sensor interface 24, upon which are rigidly mounted the laser rangefinder 21 and the inertial sensor unit 22, to form an integral assembly with its components moving concurrently.

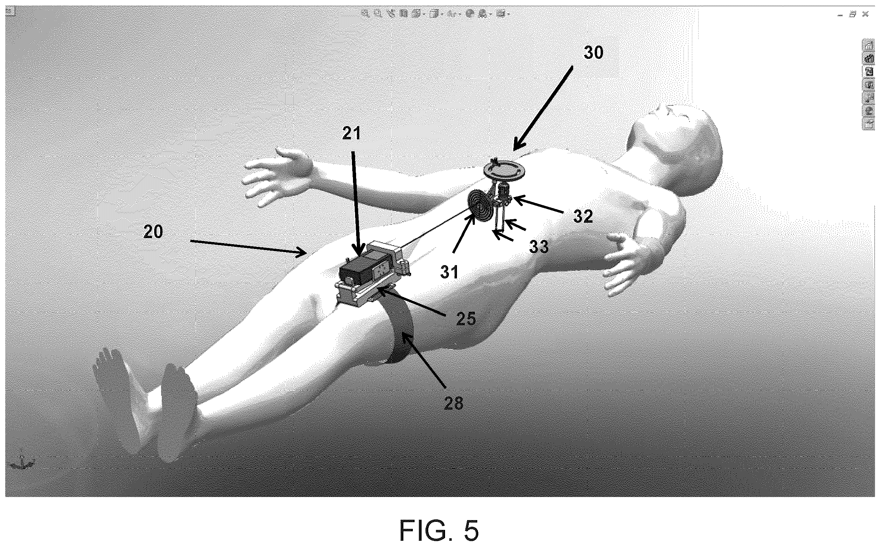

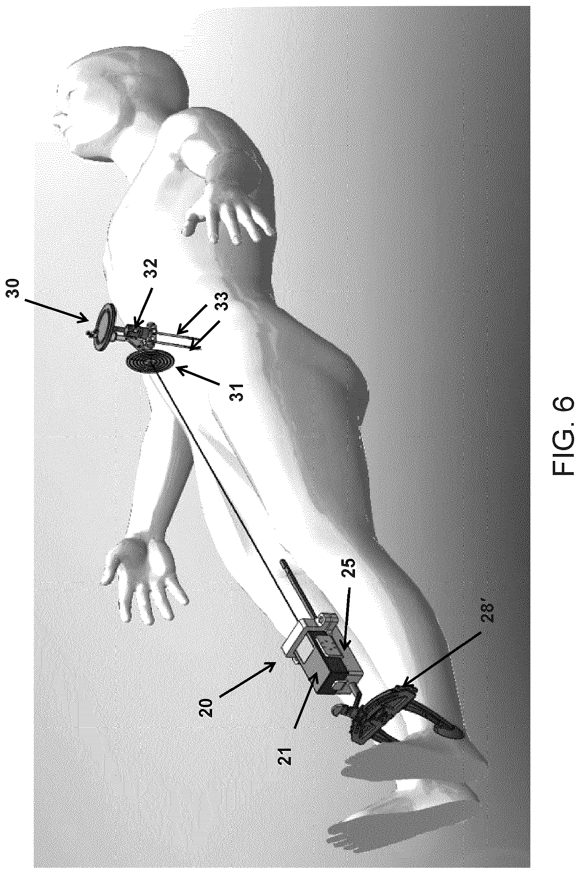

The sensor interface 24 is mounted to a base 25, with a lockable rotational degree of freedom (DOF) therebetween. The DOF is provided by hinge joint 26, and by locking feature 27. The locking feature 27 is shown as being a set screw and nut for ease of manual use, but other embodiments are possible. Referring to Fig. 5, a body attachment is a strap 28 rigidly attaching the apparatus 20 to a limb. Referring to Fig. 6, the body attachment may be a caliper 28' attaching the apparatus 20 to a limb, such as the ankle malleoli. Even though the caliper 28' secures the apparatus 20 such that the position of the apparatus 20 is fixed, an additional rotational DOF allows angular movements in yaw, such that the orientation of the laser rangefinder 21 may be adjusted to aim the beam of the laser rangefinder 21 on the board reference device 30, or directly on selected landmarks without using board device 30, depending on the application. In similar fashion to the arrangement of Fig. 2, the change in orientation will be determined by the inertial sensor unit 22 such that, once the beam illuminates the target on the board reference device 30 or directly on the selected landmarks without using board device 30, depending on the application, the 3D orientation of the apparatus 20 is recorded. The distance measurement provided by the laser rangefinder 21 is combined to the 3D orientation of the apparatus 20, to perform the trigonometric calculations to obtain leg length discrepancy and/or offset, among other possibilities.

Referring to Figs. 5 and 6, the board reference device 30 has a target board 31. The device 30 also has a base 32 to be anchored to a body portion. In the illustrated embodiment, the base 32 features a pair of pins 33 that will be fixed to a bone landmark, such as the pelvis. The pins 33 are selected as being a minimally invasive embodiment, but other options are considered, such as non-invasive solutions such as straps, adhesive. According to an embodiment, it is important that the board reference device 30 remains in the exact same position and orientation throughout the measurements (pre- and post-implanting) or that a position and orientation of the board reference device 30 may be replicated with accuracy and precision, if movement of the device 30 is not tracked. It is also contemplated to provide the device 30 with sensors, such as another of the inertial sensor unit 22. As mentioned above, the device 30 is optionally in some applications, but may assist in providing an accurate target and/or increasing visibility.

Referring to Fig. 1, the CAS processing unit 40 may be integral with the inertial sensor unit 22 (or one of them if more than one is present), as schematically and optionally shown as A, or may be as a module of a computer or portable device, among other possibilities, and thus not part of the integral pod A. A user interface(s) 40 outputs the navigation data (e.g., such as leg length discrepancy and offset), whether it be in the form of LED displays, screens, numerical displays, etc. Alternatively, if the CAS processing unit 40 is part of a pod A with the inertial sensor unit 22, the pod A may be connected to a stand-alone processing device B that would include a screen or like monitor, to provide additional display capacity and surface. By way of example, the processing device B is a wireless portable device such as a tablet in a wired or wireless communication with the pod A. The calculations and steps set forth below are not dependent on the physical arrangement between the inertial sensor unit 22 and the processing unit 40.

The processing unit 40 comprises different modules to perform the navigation and output the leg length and offset data. A surgical flow module 40A may be used in conjunction with the user interface 50 or with the processing device B to guide the operator through the steps leading to the navigation. This may entail providing a step-by-step guidance to the operator, such as what landmark distance to record next, and prompting the operator to perform actions, for instance pressing on a "record" interface that is part of the rangefinder apparatus 20 or of the interface 50 or entering data as measured using the rangefinder apparatus 20, for the system 10 to record instant orientations and distances. While this occurs throughout the surgical procedure, the prompting and interactions between the system 10 and the user will not be described in a remainder of the description, as they will implicitly occur. It is contemplated to have the surgical flow module 40A present in the processing device B, with concurrent action between the inertial sensor unit 22 and the processing device B to guide the operator during the measuring procedures detailed below, and with a communication with the operator to record the progress of the procedure.

A tracking module 40B is part of the processing unit 40. The tracking module 40B receives readings from the inertial sensor unit 22, converts these readings to useful information, i.e., the navigation data, and records the orientation of the rangefinder 21 as provided by the tracking module 40B, for each landmark distance measurement. As described above, the navigation data may be orientation data in three rotational DOFs for the rangefinder 21, at the instant distance measurements. Accordingly, the tracking module 40B may perform dead reckoning to track the inertial sensor unit 22. Dead reckoning is commonly known and documented and forms part of the common general knowledge, as using inertial sensor readings to continually calculate the orientation and velocity of a body without the need for an external reference, i.e., no signal transmission from outside of the sensor assembly is necessary, the inertial sensor unit 22 is self-contained. An initial orientation and velocity must be provided to the inertial sensor unit 22, e.g., a X-Y-Z coordinate system, after which the orientation is tracked by integrating the angular rates of gyroscope readings at each time step. Since the inertial sensor unit 22 has no need for an external reference, it may be immune to environmental factors such as magnetic fields and operate under a wide range of conditions.

The coordinate system module 40C creates the coordinate system using the orientation data produced by the tracking module 40B, and related distance measurement. The coordinate system is the virtual frame of reference in which the landmarks have coordinates, based on the readings from the rangefinder 21 and the inertial sensor unit 22. For example, the coordinate system module 40C sets a pelvic coordinate system from readings of the rangefinder 21 and the inertial sensor unit 22. The origin of the coordinate system may be arbitrary, for instance using the rangefinder 21 as origin, or superposing one of the landmarks as the origin, for simplicity.

In order to calculate and output the offset and leg length discrepancy, via the user interface 50 or processing device B, the processing unit 40 has a length calculation module 40D. The length calculation module 40D uses the coordinates of the landmarks in the coordinate system created by the coordinate system module 40C, to calculate the leg length discrepancy and/or the offset, using 3D trigonometry. The length calculation module 40D may therefore output the leg length discrepancy and offset.

Now that the system 10 has been described, a method for measuring leg length and offset is set forth. The patient may be positioned in any appropriate position, including supine decubitus and lateral decubitus, provided there is a line of sight between the rangefinder 21 and the landmarks discussed hereinafter. The rangefinder apparatus 20 is positioned in such a way that there is an unobstructed line of sight between the rangefinder 21 and the desired landmarks on the patient. For example, the rangefinder apparatus 20 with tripod 24 of Fig. 3 may be positioned adjacent to the patient. The rangefinder apparatus 20 connected to the patient by the caliper 28' in Fig. 6 may also be used with this approach.

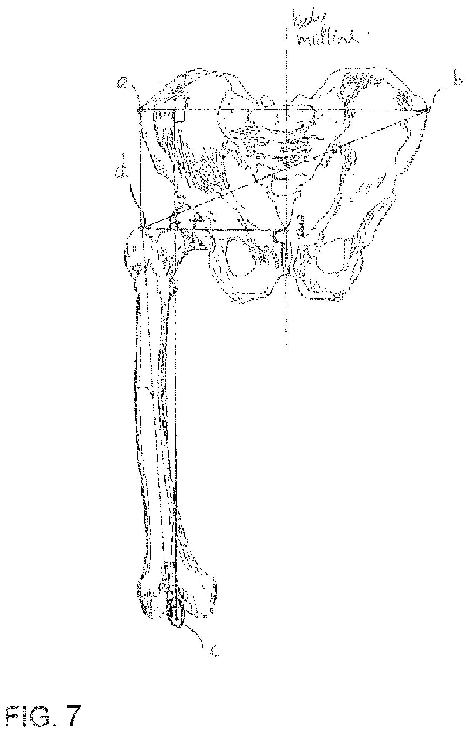

In the exemplary method, which does not form part of the present invention, involving the rangefinder apparatus 20 of Fig. 3 or of Fig. 6, laser distance measurements are performed on chosen landmarks, for example before resection, and after implanting, also referred to as preoperatively and postoperatively. The landmarks may be any appropriate landmarks representative of leg length and offset variations relative to a remainder of the body. According to an exemplary method, which does not form part of the present invention, as shown in Fig. 7, two reference landmarks are taken on the pelvis and are used as a reference for distance variations. For clarity, the pelvis and femur are shown without soft tissue. However, the reference landmarks are generally covered with soft tissue, with the exception of one femoral landmark. For example, the reference landmarks on the pelvis may be ① the left ASIS (antero-superior iliac spine), and ② the right ASIS. The ASIS are visually distinct even when covered by skin. Other pelvic landmarks may be used, for instance using added markers onto the patient's skin. For example, it may be difficult to place patients with high body-mass indexes in lateral decubitus as the ASIS may not be visible with the rangefinder 21. In such cases, other landmarks on the pelvis may be used.

Two femoral landmarks may be used, such as ③ patella for leg length calculation due to its visual distinctiveness when covered by skin, and ④ the exposed greater trochanter (i.e., not covered by soft tissue), for offset calculation. Other landmarks could be used as well, such as a random point near the greater trochanter for offset, and a random point on the tibia or the femur for leg length discrepancy, provided the landmarks are the same through the procedure.

When using the rangefinder apparatus of Fig. 3 or of Fig. 6, the instant distance measurements from the laser rangefinder 21 and corresponding orientation data from the inertial sensor unit 22 are recorded by the tracking module 40B of the CAS processing unit 40, for these four landmarks, pre- and postoperatively. The leg length and offset can then be obtained by mathematical calculations in the CAS processing unit 40 based on resolving 3D trigonometry, using the combined actions of the coordinate system module 40C and length calculation module 40D.

Referring to Fig. 7, the landmarks (2 ASISs, patella, the greater trochanter) are projected on a reference plane where the leg length and offset calculations take place. The projected landmarks are shown, where:
a, b = two ASISs projected on the reference plane;
c = patella projected on the reference plane;
d = greater trochanter projected on the reference plane;
Leg length (cf) = the distance between the lines connecting two ASISs (ab) and patella (c);
Leg offset (dg) = the distance between the body midline (i.e., midpoint between the two ASISs) and greater trochanter (d);
Leg length and offset discrepancy (Δcf, Δdg) = the difference between the preoperative and postoperative leg length and offset measurements. When the three sides of the triangles (abc, abd) are known, the triangles are resolved;
therefore, leg length (cf) and offset (dg) are calculated by the CAS processing unit 40.

The CAS processor unit 40 guides an operator in taking these landmark measurements, for instance using the surgical flow module 40A. The CAS processor unit 40 may then record sets of orientation and distance for each landmark using the tracking module 40B. The coordinate system module 40C converts the orientation and distance measurements to coordinates, using the orientation data from the tracking module 40B and the corresponding distance measurements from the rangefinder 21. The length calculation module 40D may then calculate Δcf, Δdg once all landmark coordinates are obtained pre- and postoperatively.

The length calculation module 40D may use the line a,b as a reference axis, as it is the same preoperatively and postoperatively. Therefore, when calculating leg length measurement, the sides a,b (i.e., the reference axis) of the preoperative triangle abc and of its counterpart postoperative triangle abc are superposed. Preoperative and intra- or post-operative planes, respectively including preoperative and intra- or post-operative triangles abc are then relatively rotated about the reference axis (sides a,b) for the triangles abc to be in a same plane, referred to as the reference plane (it is noted that the reference plane may be coincident with the preoperative triangle abd). When the triangles abc are in the reference plane, leg length cf may be calculated by the length calculation module 40D.

Likewise, the line a,b is used as a reference axis in the offset calculation as well. Therefore, when calculating offset, the sides a,b (i.e., the reference axis) of the preoperative triangle abd and of its counterpart postoperative triangle abd are superposed. The two triangles abd are then rotated about the reference axis (sides a,b) for the triangles abd to be in the same reference. When the triangles abd are in the reference plane, the offset dg may be calculated by the length calculation module 40D. The reference plane for the offset may be different or the same as the reference plane for the leg length calculation.

The method described above does not require that the leg be repositioned in the same plane as it was prior to resection, as the triangles abc and abd are virtually aligned in the reference plane. It is however desired that the general longitudinal leg alignment be preserved, for instance using visual or mechanical assistance to do so, or by aligning the operated leg with the non-operated leg.

When using the rangefinder apparatuses of Figs. 4, 5 and 6, the accelerometers in the inertial sensor unit 22 may be used to assist in transforming the readings to "reference plane" values, with the calculation approach of Fig. 7 being used once more. For example, the landmarks (2 ASISs, patella, the greater trochanter) are projected on the frontal plane where the leg length and offset calculations take place. This is an optional feature, but may simplify the calculations. The frontal plane is obtained by using the gravity acquired by the accelerometer in the inertial sensor unit 22, based on the assumption that the OR table plane represents the patient frontal plane when the patient is in supine decubitus.

Referring to Fig. 5, the system 10 is used to measure the leg length discrepancy. The apparatus 20 is rigidly attached to the femur by the strap 28. The distance measurement will be taken from the distal femur to the pelvis using the laser rangefinder 21. The strapped-down fixation 28 ensures the apparatus 20 is securely fastened on the distal femur with no or minimum movement when the leg undergoes movement during resection and implanting. Moreover, this strapped-down fixation 28 may ensure that the orientation of the laser rangefinder 21 follows the longitudinal axis of the patient, instead of allowing the yaw orientation adjustment of the laser rangefinder 21 relative to the longitudinal axis. As shown, the apparatus 20 has fewer DOFs of movement and cannot be fully adjusted in orientation. The board reference device 30, upon which the laser beam is to be targeted, is pinned to the iliac crest. The orientation and position of the target board 31 could be adjusted to make up for the lack of adjustments provided at the apparatus 20, relative to its base 32; therefore, the target board 31 can be aligned with the laser beam along the patient longitudinal axis. When the OR table is leveled, it is again assumed that the OR table plane represents the patient frontal plane when the patient is in supine. The distance from the laser rangefinder 21 to the target board 31 and the inclination of the laser rangefinder 21 with respect to horizontal can be obtained from its readings. Then, the distance projected on the horizontal plane can be calculated based on simple trigonometry. As the distance measurement direction (e.g., the laser beam) is aligned with the patient longitudinal axis, the projected distance measurement in the patient frontal plane represents the leg length. Taking the leg length measurement prior to and after the surgery provides leg length discrepancy. The target board 31 may have a scale thereon, for the lateral movement to be noted before and after surgery to obtain a visual reading of offset.

Another exemplary method, which does not form part of the present invention, may include positioning the rangefinder apparatus 20 at an output end of a robotic arm. In such a case, the rangefinder apparatus 20 may be with or without an inertial sensor unit 22. The CAS processing unit 40 may use orientation data obtained from the control of the robotic arm instead of data from an inertial sensor unit. For example, the robotic arm may be a serial arm with encoders at joints to determine the position and orientation of the rangefinder apparatus 20.

Therefore, the system 10 may be used to execute a method for measuring a length variation between body portions in computer-assisted surgery, between a preoperative condition and intra- or post-operative condition. According to the method, a distance to at least one reference landmark on a first bone and orientation data from the rangefinder apparatus 20 is obtained in a known position relative to a second bone. The rangefinder apparatus 20 is tracked in a virtual coordinate system using the orientation data. Coordinates in the virtual coordinate system of the at least one reference landmark are determined using the distance and the orientation data. A length is measured between the bones using the coordinates. The length variation between the body portions is calculated and output by using the length obtained from a preoperative condition and said length obtained from an intra- or post-operative condition. The distance may be obtained with the rangefinder apparatus 20 being fixed to the second body portion in the known position.

## Claims

1. A system (10) for measuring a length variation between body portions in computer-assisted surgery between a preoperative condition and an intra- or post-operative condition comprising:
a rangefinder apparatus (20) comprising a rangefinder (21) configured to measure its distance to at least one reference landmark on at least a first body portion of a patient from a known position relative to a second body portion by emitting light on the at least one reference landmark and optically detecting its distance from the light on the at least one reference landmark, a support (23) including at least one joint allowing at least one rotational degree of freedom of movement of the rangefinder (21) to point to the at least one reference landmark, and an inertial sensor unit (22) connected to the rangefinder (21) to produce orientation data for the rangefinder (21); and
a computer-assisted surgery processing unit (40) having
a tracking module for tracking the rangefinder (21) in a virtual coordinate system using the orientation data,
a coordinate system module (40C) for determining coordinates in the virtual coordinate system of the at least one reference landmark using at least one combination of the distance and the orientation data, the orientation data being instant to the distance in the at least one combination; and
a length calculation module (40D) for measuring a preoperative length between the body portions using the coordinates of light emitted on the at least one reference landmark in a preoperative condition, and for measuring an intra- or post-operative length between the body portions using the coordinates of light emitted on the at least one reference landmark in an intra- or post-operative condition, wherein the length calculation module (40D) is configured to calculate and output the length variation between the body portions by using said preoperative length obtained from the preoperative condition and said intra- or post-operative length obtained from the intra-or post-operative condition.

2. The system according to claim 1, wherein the support (23) is configured to fix the rangefinder (21) to the second body portion in the known position.

3. The system according to claim 2, wherein the second body portion is an elongated bone, and wherein the rangefinder (21) is configured to have its distance measurement direction aligned with a longitudinal axis of the elongated bone.

4. The system according to any one of claims 1 and 2, wherein the length calculation module (40D) is configured to project said lengths obtained from the preoperative condition and from the intra- or post-operative condition on a reference plane to calculate the length variation.

5. The system according to claim 4, wherein the patient is in a supine position and wherein the reference plane is parallel to a plane of a table supporting the patient, the reference plane is configured to determine by the coordinate system module (40C) using readings from the inertial sensor unit (22).

6. The system according to any one of claims 2 to 5, wherein the at least one reference landmark is a board reference device (30) including a target board secured to the first body portion.

7. The system according to claim 6, wherein the target board has a visual scale thereon to indicate a lateral displacement of the second body portion relative to the first body portion between the preoperative condition and the intra- or post-operative condition.

8. The system according to any one of claims 2 to 7, wherein the first body portion is a pelvis, and the second body portion is a femur, the computer-assisted surgery including alterations to a hip joint.

9. The system according to claim 1, wherein the rangefinder apparatus (20) is configured to be adjacent to the body portions, wherein the tracking module (40B) is configured to track the rangefinder (21) to the known position using the distance and orientation of the rangefinder (21) relative to at least one reference landmark on the second body portion.

10. The system according to claim 9, wherein the first body portion is an elongated bone and the second body portion is another bone jointed to the elongated bone, the computer-assisted surgery including alterations to a joint between the elongated bone and the other bone, and wherein the known position of the rangefinder (21) relative to the second body portion comprises two of the at least one reference landmark on the second body portion.

11. The system according to claim 10, wherein the length calculation module (40D) is configured to form a preoperative plane with the coordinates of the at least one reference landmark on the first body portion and of the two reference landmarks on the second body portion obtained from the preoperative condition, and is configured to form an intra- or post-operative plane with the coordinates of the at least one reference landmark on the first body portion and of the two reference landmarks on the second body portion obtained from the intra- or post-operative condition, the length calculation module (40D) is configured to rotate at least one of the planes about an axis passing through the two reference landmarks on the second body portion to obtain a parallel relation between the planes, to calculate the length variation from the parallel relation.

12. The system according to claim 11, wherein the length calculation module (40D) determines a lateral position variation of the at least one reference landmark on the first body portion in a direction parallel to the axis passing through the two reference landmarks on the second body portion, to calculate an offset of the first body portion.

13. The system according to any one of claims 9 to 12, the first body portion is a femur, and the second body portion is a pelvis, the computer-assisted surgery including alterations to a hip joint between the femur and the pelvis.

## Patentansprüche

1. System (10) zum Messen einer Längenvariation zwischen Körperteilen bei der computergestützten Chirurgie zwischen einem präoperativen Zustand und einem intra- oder postoperativen Zustand, umfassend:
eine Entfernungsmesservorrichtung (20), die einen Entfernungsmesser (21) umfasst, der konfiguriert ist, um seinen Abstand zu mindestens einem Referenzpunkt auf mindestens einem ersten Körperteil eines Patienten aus einer bekannten Position relativ zu einem zweiten Körperteil zu messen, indem Licht auf den mindestens einen Referenzpunkt emittiert wird und optisch dessen Abstand von dem Licht auf dem mindestens einen Referenzpunkt erfasst wird, eine Halterung (23) mit mindestens einem Gelenk, das mindestens einen Rotationsfreiheitsgrad der Bewegung des Entfernungsmessers (21) ermöglicht, um auf den mindestens einen Referenzpunkt zu zeigen, und eine Trägheitssensoreinheit (22), die mit dem Entfernungsmesser (21) verbunden ist, um Orientierungsdaten für den Entfernungsmesser (21) zu erzeugen; und
eine computergestützte Chirurgieverarbeitungseinheit (40) mit
ein Verfolgungsmodul zum Verfolgen des Entfernungsmessers (21) in einem virtuellen Koordinatensystem unter Verwendung der Orientierungsdaten,
ein Koordinatensystemmodul (40C) zum Bestimmen von Koordinaten in dem virtuellen Koordinatensystem des mindestens einen Referenzpunkts unter Verwendung mindestens einer Kombination des Abstands und der Orientierungsdaten, wobei die Orientierungsdaten in der mindestens einen Kombination unmittelbar zu dem Abstand sind; und
ein Längenberechnungsmodul (40D) zum Messen einer präoperativen Länge zwischen den Körperteilen unter Verwendung der Koordinaten des Lichts, das in einem präoperativen Zustand auf den mindestens einen Referenzpunkt emittiert wird, und zum Messen einer intra- oder postoperativen Länge zwischen den Körperteilen unter Verwendung der Koordinaten des Lichts, das in einem intra- oder postoperativen Zustand auf den mindestens einen Referenzpunkt emittiert wird, wobei das Längenberechnungsmodul (40D) konfiguriert ist, um die Längenvariation zwischen den Körperteilen zu berechnen und auszugeben, indem die aus dem präoperative Zustand erhaltene präoperativen Länge und die aus dem intra- oder postoperativen Zustand erhaltene intra-oder postoperative Länge verwendet wird.

2. System nach Anspruch 1, wobei die Halterung (23) konfiguriert ist, um den Entfernungsmesser (21) an dem zweiten Körperteil in der bekannten Position zu befestigen.

3. System nach Anspruch 2, wobei der zweite Körperteil ein verlängerter Knochen ist und wobei der Entfernungsmesser (21) so konfiguriert ist, dass seine Entfernungsmessrichtung mit einer Längsachse des verlängerten Knochens ausgerichtet ist.

4. System nach einem der Ansprüche 1 und 2, wobei das Längenberechnungsmodul (40D) konfiguriert ist, um die aus dem präoperativen Zustand und aus dem intra- oder postoperativen Zustand erhaltenen Längen auf eine Referenzebene zu projizieren, um die Längenvariation zu berechnen.

5. System nach Anspruch 4, wobei sich der Patient in Rückenlage befindet und wobei die Referenzebene parallel zu einer Ebene eines den Patienten tragenden Tisches ist, wobei die Referenzebene konfiguriert ist, um durch das Koordinatensystemmodul (40C) unter Verwendung von Ablesungen der Trägheitssensoreinheit (22) zu bestimmen.

6. System nach einem der Ansprüche 2 bis 5, wobei der mindestens eine Referenzpunkt eine Platten-Referenzvorrichtung (30) ist, die eine an dem ersten Körperteil befestigte Zielplatte umfasst.

7. System nach Anspruch 6, wobei die Zielplatte eine visuelle Skala darauf aufweist, um eine seitliche Verschiebung des zweiten Körperteils relativ zum ersten Körperteil zwischen dem präoperativen Zustand und dem intra- oder postoperativen Zustand anzuzeigen.

8. System nach einem der Ansprüche 2 bis 7, wobei der erste Körperteil ein Becken ist und der zweite Körperteil ein Oberschenkelknochen ist, wobei die computergestützte Chirurgie Veränderungen an einem Hüftgelenk umfasst.

9. System nach Anspruch 1, wobei die Entfernungsmesservorrichtung (20) konfiguriert ist, um benachbart zu den Körperteilen zu sein, wobei das Verfolgungsmodul (40B) konfiguriert ist, um den Entfernungsmesser (21) zu der bekannten Position unter Verwendung des Abstands und der Orientierung des Entfernungsmessers (21) relativ zu mindestens einem Referenzpunkt auf dem zweiten Körperteil zu verfolgen.

10. System nach Anspruch 9, wobei der erste Körperteil ein verlängerter Knochen ist und der zweite Körperteil ein anderer Knochen ist, der mit dem verlängerten Knochen verbunden ist, wobei die computergestützte Chirurgie Veränderungen an einer Verbindung zwischen dem verlängerten Knochen und dem anderen Knochen umfasst, und wobei die bekannte Position des Entfernungsmessers (21) relativ zu dem zweiten Körperteil zwei des mindestens einen Referenzpunkts auf dem zweiten Körperteil umfasst.

11. System nach Anspruch 10, wobei das Längenberechnungsmodul (40D) konfiguriert ist, um eine präoperative Ebene mit den Koordinaten des mindestens einen Referenzpunkts auf dem ersten Körperteil und der zwei Referenzpunkte auf dem zweiten Körperteil zu bilden, die aus dem präoperativen Zustand erhalten werden, und konfiguriert ist, um eine intra- oder postoperative Ebene mit den Koordinaten des mindestens einen Referenzpunkts auf dem ersten Körperteil und der zwei Referenzpunkte auf dem zweiten Körperteil zu bilden, die aus dem intra- oder postoperativen Zustand erhalten werden, das Längenberechnungsmodul (40D) konfiguriert ist, um mindestens eine der Ebenen um eine Achse zu drehen, die durch die zwei Referenzpunkte auf dem zweiten Körperteil verläuft, um eine parallele Beziehung zwischen den Ebenen zu erhalten, um die Längenvariation aus der parallelen Beziehung zu berechnen.

12. System nach Anspruch 11, wobei das Längenberechnungsmodul (40D) eine seitliche Positionsvariation des mindestens einen Referenzpunkts auf dem ersten Körperteil in einer Richtung parallel zu der Achse bestimmt, die durch die zwei Referenzpunkte auf dem zweiten Körperteil verläuft, um einen Versatz des ersten Körperteils zu berechnen.

13. System nach einem der Ansprüche 9 bis 12, wobei der erste Körperteil ein Oberschenkelknochen ist und der zweite Körperteil Becken ein ist, wobei die computergestützte Chirurgie Veränderungen an einem Hüftgelenk zwischen dem Oberschenkelknochen und dem Becken umfasst.

## Revendications

1. Système (10) destiné à mesurer une variation de longueur entre des parties de corps en chirurgie assistée par ordinateur entre un état préopératoire et un état intra- ou post-opératoire comprenant :
un appareil télémètre (20) comprenant un télémètre (21) conçu pour mesurer sa distance à jusqu'à au moins un repère de référence sur au moins une première partie de corps d'un patient à partir d'une position connue par rapport à une seconde partie de corps en émettant de la lumière sur le au moins un repère de référence et détectant optiquement sa distance à partir de la lumière sur le au moins un repère de référence, un support (23) comprenant au moins une articulation permettant au moins un degré de liberté de mouvement en rotation du télémètre (21) pour pointer vers le au moins un repère de référence, et une unité de capteur d'inertie (22) reliée au télémètre (21) pour produire des données d'orientation pour le télémètre (21) ; et
une unité de traitement de chirurgie assistée par ordinateur (40) possédant
un module de suivi destiné à suivre le télémètre (21) dans un système de coordonnées virtuelles à l'aide des données d'orientation,
un module de système de coordonnées (40C) destiné à déterminer des coordonnées dans le système de coordonnées virtuel du au moins un repère de référence à l'aide d'au moins une combinaison de la distance et des données d'orientation, les données d'orientation étant instantanées par rapport à la distance dans la au moins une combinaison ; et
un module de calcul de longueur (40D) destiné à mesurer une longueur préopératoire entre les parties de corps à l'aide des coordonnées de lumière émise sur le au moins un repère de référence dans un état préopératoire, et destiné à mesurer une longueur intra- ou post-opératoire entre les parties de corps à l'aide des coordonnées de lumière émise sur le au moins un repère de référence dans un état intra- ou post-opératoire, ledit module de calcul de longueur (40D) étant conçu pour calculer et délivrer en sortie la variation de longueur entre les parties de corps à l'aide de ladite longueur préopératoire obtenue à partir de l'état préopératoire et ladite longueur intra- ou post-opératoire obtenue à partir de l'état intra- ou post-opératoire.

2. Système selon la revendication 1, ledit support (23) étant conçue pour fixer le télémètre (21) à la seconde partie de corps dans la position connue.

3. Système selon la revendication 2, ladite seconde partie de corps étant un os allongé, et ledit télémètre (21) étant conçu pour posséder sa direction de mesure de distance alignée avec un axe longitudinal de l'os allongé.

4. Système selon l'une quelconque des revendications 1 et 2, ledit module de calcul de longueur (40D) étant conçu pour projeter lesdites longueurs obtenues à partir de l'état préopératoire et à partir de l'état intra- ou post-opératoire sur un plan de référence pour calculer la variation de longueur.

5. Système selon la revendication 4, ledit patient étant dans une position couchée et ledit plan de référence étant parallèle à un plan d'une table supportant le patient, ledit plan de référence étant configuré pour être déterminé par le module de système de coordonnées (40C) à l'aide des lectures provenant de l'unité de capteur d'inertie (22).

6. Système selon l'une quelconque des revendications 2 à 5, ledit au moins un point de repère de référence étant un dispositif de référence de plateau (30) comprenant un plateau cible fixé à la première partie de corps.

7. Système selon la revendication 6, ledit panneau cible possédant une échelle visuelle sur celui-ci pour indiquer un déplacement latéral de la seconde partie de corps par rapport à la première partie de corps entre l'état pré-opératoire et la condition intra- ou post-opératoire.

8. Système selon l'une quelconque des revendications 2 à 7, ladite première partie de corps étant un bassin et ladite seconde partie de corps étant un fémur, la chirurgie assistée par ordinateur comprenant des modifications d'une articulation de hanche.

9. Système selon la revendication 1, ledit appareil télémètre (20) étant configuré pour être adjacent aux parties de corps, ledit module de suivi (40B) étant configuré pour suivre le télémètre (21) jusqu'à la position connue à l'aide de la distance et de l'orientation du télémètre (21) par rapport à au moins un repère de référence sur la seconde partie de corps.

10. Système selon la revendication 9, ladite première partie de corps étant un os allongé et ladite seconde partie de corps étant un autre os joint à l'os allongé, la chirurgie assistée par ordinateur comprenant des modifications d'une articulation entre l'os allongé et l'autre os, et ladite position connue du télémètre (21) par rapport à la seconde partie de corps comprenant deux du au moins un repère de référence sur la seconde partie de corps.

11. Système selon la revendication 10, ledit module de calcul de longueur (40D) étant conçu pour former un plan préopératoire avec les coordonnées du au moins un repère de référence sur la première partie de corps et des deux repères de référence sur la seconde partie de corps obtenues à partir de l'état préopératoire, et étant conçu pour former un plan intra- ou post-opératoire avec les coordonnées du au moins un repère de référence sur la première partie de corps et des deux repères de référence sur la seconde partie de corps obtenues à partir de l'état intra- et post-opératoire, ledit module de calcul de longueur (40D) étant configuré pour faire tourner au moins l'un des plans autour d'un axe passant par les deux repères de référence sur la seconde partie de corps pour obtenir une relation parallèle entre les plans, pour calculer la variation de longueur à partir de la relation parallèle.

12. Système selon la revendication 11, ledit module de calcul de longueur (40D) déterminant une variation de position latérale du au moins un repère de référence sur la première partie de corps dans une direction parallèle à l'axe passant par les deux repères de référence sur la seconde partie de corps, pour calculer un décalage de la première partie de corps.

13. Système selon l'une quelconque des revendications 9 à 12, la première partie de corps est un fémur et la seconde partie de corps est un bassin, la chirurgie assistée par ordinateur comprenant des modifications d'une articulation de hanche entre le fémur et le bassin.
